# EUROPEAN PATENT APPLICATION

(11) **EP 2 315 015 A1**
(43) Date of publication of application: **27.04.2011**
(21) Application number: 09800372.6
(22) Date of filing: 17.07.2009
(51) Int. Cl.: G01N 27/447

(54) **ANALYSIS DEVICE BY CAPILLARY ELECTROPHORESIS METHOD**

(30) Priority: 22.07.2008 JP 2008188828
(71) Applicant: ARKRAY, Inc., Kyoto-shi, Kyoto 601-8045 (JP)
(72) Inventor: SUGIYAMA, Koji, Minami-ku, Kyoto-shi, Kyoto 601-8045 (JP); HIGASHIISOKAWA, Yukio, Kyoto-shi, Kyoto 615-0937 (JP); NAKAYAMA, Yusuke, Minami-ku, Kyoto-shi, Kyoto 601-8045 (JP)
(74) Representative: Piésold, Alexander James
(86) International application number: PCT/JP2009/063009
(87) International publication number: WO 2010/010858

(57) **Abstract**

A capillary electrophoresis analysis apparatus, for analyzing protein in blood by a capillary electrophoresis method, is provided that allows the whole apparatus to be small, operation to be simple, manufacturing cost to be inexpensive, and analysis to be performed highly accurately.

A capillary electrophoresis analysis apparatus of the present invention is one for analyzing protein in blood by a capillary electrophoresis method. The capillary electrophoresis analysis apparatus comprises an electrophoresis chip, a voltage application unit, and an absorbance measurement unit. The electrophoresis chip comprises a substrate, a plurality of liquid reservoirs, and a capillary channel. The voltage application unit comprises an electrode. The plural liquid reservoirs are formed in the substrate. The plural liquid reservoirs are in communication with one another via the capillary channel. The capillary channel includes a capillary channel for sample analysis.

## Description

### Technical Field

The present invention relates to capillary electrophoresis analysis apparatuses for analyzing protein in blood by capillary electrophoresis methods.

### Background Art

Among proteins in blood, there are some which vary their concentration or the like due to diseases, and are used as indicators in the diagnosis of diseases. For example, glutamic oxalacetic transaminase (GOT) is an indicator of hepatitis or the like, serum amylase is an indicator of pancreatitis or the like, and albumin/globulin ratio is an indicator of nephrosis or the like. These proteins in blood are analyzed using cellulose acetate membrane electrophoresis methods or the like, for example. Further, hemoglobin (Hb) which is protein in blood includes hemoglobin A (HbA), hemoglobin F (HbF), hemoglobin S (HbS), and glycated hemoglobin, which is a glycated product of these hemoglobin. Among them, hemoglobin S (HbS) is abnormal hemoglobin in which 6^{th} glutamic acid of β chain is substituted to valine, and is an indicator in the diagnosis of sickle-cell anemia. The glycated hemoglobin is hemoglobin that is reacted with glucose in blood, and is used as an indicator in diagnosis and treatment of diabetes. The glycated hemoglobin includes hemoglobin A1a (HbAla), hemoglobin Alb (HbA1b), hemoglobin A1e (HbA1c), GHbLys, etc. The hemoglobin A1c is glycated hemoglobin whose β chain N-terminal valine is glycated. The hemoglobin A1c is an indicator that reflects glucose level of the past few months and is an examination item of routine physical examinations. As described above, proteins in blood are important indicators of various diseases. Hence, the development of analysis apparatuses for protein in blood, which are applicable in laboratory tests or the like, low in running costs, and reduced in size, is desired.

Examples of the method of measuring hemoglobin in blood include immunological methods, enzymatic methods, affinity chromatography methods, HPLC methods, capillary electrophoresis methods, and the like. Since the immunological methods and the enzymatic methods can be applied to autoanalysis apparatuses, they have advantages of handling large numbers of specimens. However, the immunological methods and the enzymatic methods lack measurement accuracy. Further, in separation principle, the affinity chromatography methods have low specificity relative to a glycated valine of a β chain N-terminal, and glycated lysine in Hb molecule is to be contained in a measurement value. Therefore, the measurement accuracy of hemoglobin A1c in the affinity chromatography methods is low. Further, the HPLC methods are used widely as methods of measuring hemoglobin (for example, Patent Document 1). However, the HPLC methods require large and expensive special apparatuses and it is difficult to reduce size and cost of the apparatuses. For applying to the group examinations and the like, analysis apparatuses of hemoglobin are required to be downsized. However, as described above, it is difficult for the HPLC methods to satisfy this requirement.

[Patent Document 1] JP3429709 B

### Disclosure of Invention

Hence, the present invention is intended to provide a capillary electrophoresis analysis apparatus, for analyzing protein in blood by a capillary electrophoresis method, that allows the whole apparatus to be small, operation to be simple, manufacturing cost to be inexpensive, and analysis to be performed highly accurately.

In order to achieve the aforementioned object, the capillary electrophoresis analysis apparatus of the present invention is a capillary electrophoresis analysis apparatus for analyzing protein in blood by a capillary electrophoresis method that comprises
an electrophoresis chip, a voltage application unit, and an absorbance measurement unit,
the electrophoresis chip comprises a substrate, a plurality of liquid reservoirs, and a capillary channel,
the plurality of liquid reservoirs are formed in the substrate,
the plurality of liquid reservoirs are in communication with one another via the capillary channel,
the capillary channel includes a capillary channel for sample analysis,
the voltage application unit comprises an electrode,
the capillary channel for sample analysis is filled with an electrophoresis running buffer,
a sample containing the protein in blood to be analyzed is introduced into the capillary channel for sample analysis that is filled with the electrophoresis running buffer,
the sample is subjected to an electrophoresis by applying voltage to the electrode, and
an absorbance of the protein in blood in the sample subjected to the electrophoresis is measured by the absorbance measurement unit.

The capillary electrophoresis analysis apparatus of the present invention allows the whole apparatus to be small, operation to be simple, manufacturing cost to be inexpensive, and protein in blood to be analyzed highly accurately. Particularly, the capillary electrophoresis analysis apparatus of the present invention is suitable for micro total analysis systems (µTAS).

### Brief Description of Drawings

FIG. 1A is a plane view showing an example of an electrophoresis chip contained in the capillary electrophoresis analysis apparatus of the present invention. FIG. 1B is a cross-sectional view viewed in the direction of line I-I of the electrophoresis chip shown in FIG. 1A.
FIG. 2 is a schematic view showing an example of the capillary electrophoresis analysis apparatus of the present invention.
FIG. 3A is a plane view showing another example of an electrophoresis chip in the capillary electrophoresis analysis apparatus of the present invention. FIG. 3B is a cross-sectional view of the electrophoresis chip shown in FIG. 3A viewed in the direction of line I-I. FIG. 3C is a cross-sectional view of the electrophoresis chip shown in FIG. 3A viewed in the direction of line II-II.
FIG. 4A is a plane view showing yet another example of an electrophoresis chip in the capillary electrophoresis analysis apparatus of the present invention. FIG. 4B is a perspective view of the electrophoresis chip shown in FIG. 4A.
FIG. 5 is a schematic view showing another example of the capillary electrophoresis analysis apparatus of the present invention.

### Description of the Embodiments

Preferably, with respect to the capillary electrophoresis analysis apparatus of the present invention, in the capillary channel for sample analysis, a cross-sectional shape perpendicular to a channel direction is circular or rectangular,
in a case of circular, a diameter thereof is in a range of 25 µm to 100 µm, and in a case of rectangular, a width thereof is in a range of 25 µm to 100 µm and a depth thereof is in a range of 25 µm to 100 µm.

Preferably, in the capillary electrophoresis analysis apparatus of the present invention, the voltage application unit can adjust the level of voltage when applying the voltage.

Preferably, in the capillary electrophoresis analysis apparatus of the present invention, a spectroscopic method of the absorbance measurement unit is a pre-spectroscopic method.

In the capillary electrophoresis analysis apparatus of the present invention, a measurement wave length of the absorbance is preferably in a range of 260 nm to 300 nm or 380 nm to 450 nm, and more preferably in a range of 400 nm to 430 nm.

Preferably, the capillary electrophoresis analysis apparatus of the present invention further comprises at least one of a quantitative dispensing unit and a stirring unit.

Preferably, the capillary electrophoresis analysis apparatus of the present invention further comprises a liquid sending unit, and at least one of the electrophoresis running buffer and the sample is introduced into the capillary channel by a liquid sending unit.

Preferably, the capillary electrophoresis analysis apparatus of the present invention further comprises a stray light removing unit.

Preferably, the capillary electrophoresis analysis apparatus of the present invention further comprises a position adjustment unit, and at least one of a position of the electrophoresis chip and a position of the absorbance measurement unit is adjusted by the position adjustment unit.

Preferably, the capillary electrophoresis analysis apparatus of the present invention further comprises a contaminant removal unit, and contaminants in the sample can be removed by the contaminant removal unit.

Preferably, the contaminant removal unit of the capillary electrophoresis analysis apparatus is a pre-filter.

Preferably, the capillary electrophoresis analysis apparatus of the present invention further comprises an air vent unit and a detecting point on the capillary channel for sample analysis irradiated with light of a specific wavelength by the absorbance measurement unit, and the air vent unit is arranged between the detecting point and the liquid reservoir in which the sample is introduced or stored.

Preferably, the capillary electrophoresis analysis apparatus of the present invention further comprises the electrophoresis running buffer.

Preferably, the capillary electrophoresis analysis apparatus of the present invention further comprises a diluent.

Preferably, in the capillary electrophoresis analysis apparatus of the present invention, the protein in blood serving as a measurement item is hemoglobin.

Preferably, in the capillary electrophoresis analysis apparatus of the present invention, the hemoglobin is at least one selected from the group consisting of normal hemoglobin, glycated hemoglobin, modified hemoglobin, variant hemoglobin, and fetal hemoglobin.

Preferably, in the capillary electrophoresis analysis apparatus of the present invention, the hemoglobin is at least one selected from the group consisting of hemoglobin A1c, hemoglobin F, hemoglobin A2, hemoglobin S, and hemoglobin C.

Preferably, in the capillary electrophoresis analysis apparatus of the present invention, a concentration of the hemoglobin is measured. Preferably, in the capillary electrophoresis analysis apparatus of the present invention, the hemoglobin is hemoglobin A1c.

Preferably, in the capillary electrophoresis analysis apparatus of the present invention, the sample is a sample of blood which is subjected to a hemolysis treatment, and the protein in blood is hemoglobin.

Preferably, in the capillary electrophoresis analysis apparatus of the present invention, the hemolysis treatment is at least one selected from the group consisting of a surfactant treatment, an osmotic pressure treatment, and an sonication treatment.

Next, the present invention is explained in details.

As described above, the capillary electrophoresis analysis apparatus of the present invention is applicable as long as it comprises an electrophoresis chip, a voltage application unit, and an absorbance measurement unit which will be described below, and other configurations are not particularly limited. As described later, for example, the capillary electrophoresis analysis apparatus may further comprise a quantitative dispensing unit, a stirring unit, a liquid sending unit, a stray light removing unit, a position adjustment unit, a contaminant removal unit, an air vent unit, and the like.

The size of the capillary electrophoresis analysis apparatus is not particularly limited. For example, the maximum width of the whole apparatus is in the range of 10 to 100 cm, the maximum depth is in the range of 10 to 100 cm, and the maximum height is in the range of 5 to 100 cm. The size of each unit of the capillary electrophoresis analysis apparatus is also not particularly limited.

### < electrophoresis chip >

As described above, the electrophoresis chip of the present invention comprises a substrate, a plurality of liquid reservoirs, and a capillary channel.

The size of the electrophoresis chip in the capillary electrophoresis analysis apparatus is not particularly limited. For example, the maximum length thereof is in the range of 10 mm to 100 mm, the maximum width thereof is in the range of 10 mm to 60 mm, and the maximum thickness thereof is in the range of 0.3 mm to 5 mm. The maximum length of the electrophoresis chip is the length of the portion that is longest in the longitudinal direction of the electrophoresis chip. The maximum width of the electrophoresis chip is the length of the portion that is longest in the short side direction of the electrophoresis chip. The maximum thickness of the electrophoresis chip is the length of the portion that is longest in the direction (thickness direction) perpendicular to both the longitudinal direction and the short side direction of the electrophoresis chip.

The electrophoresis chip of the present invention is applicable as long as it comprises a substrate, a plurality of liquid reservoirs, and a capillary channel, and other components are not particularly limited. The electrophoresis chip may be an electrophoresis chip comprising a blood collection mechanism or an electrophoresis chip combined with a lancet.

The electrophoresis chip of the present invention may include one piece of substrate or may include more than one pieces of substrate. In the latter case, for example, the electrophoresis chip of the present invention includes an upper substrate and a lower substrate, which are laminated together. The material of the substrate is not particularly limited, and examples thereof include a glass material, a polymeric material, etc. The glass material is not particularly limited, and examples thereof include synthetic silica glass, fused silica, borosilicate glass, etc. The polymeric material is not particularly limited, and examples thereof include cycloolefin polymer (COP), polycarbonate (PC), polydimethylsiloxane (PDMS), polystyrene (PS), polylactic acid (PLA), polyethylene (PE), polytetrafluoroethylene (PTFE), polyetheretherketone (PEEK), an acrylic resin such as polymethylmethacrylate (PMMA), etc.

In the electrophoresis chip of the present invention, the liquid reservoir is applicable as long as it is formed in the substrate, and the form thereof is not particularly limited. Examples of the form of the liquid reservoir include a concave portion provided at the substrate and a space portion provided in the substrate. In the former case, for example, the liquid reservoir can be formed by forming the concave portion in the thickness direction of the substrate. Further, when the upper substrate and the lower substrate are provided as described above, for example, one of the substrates, in which a through-hole is provided, may be laminated onto the other of the substrates. By laminating the one of the substrates having the through-hole onto the other of the substrates, an opening of the through-hole is sealed at one end, and thereby the concave portion serving as the liquid reservoir is formed in a laminated body composed of the both substrates. On the other hand, in the latter case, for example, the concave portion is formed at at least one of the substrates and the both substrates may be laminated such that the surface, on which the concave portion is formed, is faced to the other one of the substrates. Due to this lamination, an opening of the concave portion of one of the substrates is sealed with the other one of the substrates, and thereby the space serving as the liquid reservoir is formed in the laminated body.

The form of the liquid reservoir is not particularly limited and examples thereof include a cylinder, a quadrangular prism, a quadrangular pyramid, a cone, and the like. The form of each liquid reservoir in the substrate may all be the same or may each be different, and it is not particularly limited. Further, the volume of each liquid reservoir is not particularly limited and is, for example, in the range of 1 mm³ to 1000 mm³, and preferably in the range of 10mm³ to 100mm³. The volume of each liquid reservoir may all be the same or may each be different, and it is not particularly limited.

In the present invention, for example, the liquid reservoir means a portion, in which liquid can be introduced or stored, and it does not always require that the liquid is actually introduced or stored therein. In the electrophoresis chip, the number of liquid reservoirs is not limited, for example. Further, a predetermined liquid reservoir may serve as more than one liquid reservoir. Examples of the liquid reservoir include a liquid reservoir for reagent, in which a reagent can be introduced or stored, a liquid reservoir for sample, in which a sample can be introduced or stored, a liquid reservoir for liquid waste, in which liquid waste is introduced or stored, a liquid reservoir for mixing, in which the sample and the reagent are mixed, and the like. In a condition where the electrophoresis chip comprises the blood collection mechanism or the lancet, the liquid reservoir for sample may be in communication with the blood collection mechanism or the lancet. In this case, the sample can be introduced into the liquid reservoir for sample from the blood collection mechanism or the lancet.

In the present invention, liquid that can be introduced or stored in the electrophoresis chip is not particularly limited, and examples thereof include a sample and a reagent. The reagent is not particularly limited, and examples thereof include an electrophoresis running buffer, an analytical reagent, a diluent, a washing liquid, etc.

In the electrophoresis chip, the form of the capillary channel is not particularly limited as long as the liquid reservoirs are in communication with one another. The capillary channel may be formed in the substrate or may be a capillary tube embedded in the substrate. In the former case, for example, a groove serving as a channel is formed on the surface of the substrate. By coating an upper part of the groove with a sealing or the like, the capillary channel may be formed. Alternatively, the groove may be formed at one of the substrates and the substrates may be laminated such that the surface, on which the groove is formed, of the one of the substrates is faced to the other one of the substrates.

The cross-sectional shape of the capillary channel is not particularly limited, and examples thereof include circular, ellipsoidal, rectangular, polygonal, etc. The diameter of the capillary channel is not particularly limited, and is, for example, in the range of 1 µm to 1000 µm, and preferably in the range of 10 µm to 200 µm. In a case where the cross-sectional shape perpendicular to the capillary channel direction is circular, the diameter of the capillary channel is the diameter of a circle. Further, in a case where the cross-sectional shape perpendicular to the capillary channel direction is not circular, the diameter of the capillary channel is, for example, the diameter of a circle having an area that corresponds to the cross sectional area of a portion having the largest cross-sectional area, the dimension of the longest line that connects two points on the circumference of the cross section, and the like.

In a case where the cross-sectional shape perpendicular to the capillary channel direction is rectangular, for example, the width thereof is in the range of 1 µm to 1000 µm and the depth thereof is in the range of 1 µm to 1000 µm, and preferably the width thereof is in the range of 10 µm to 200 µm and the depth thereof is in the range of 10 µm to 200 µm.

The length of the capillary channel is not particularly limited and is, for example, in the range of 0.5 cm to 15 cm, and preferably in the range of 1 cm to 5cm.

As described above, the capillary channel includes a capillary channel for sample analysis. In the present invention, a sample is introduced into the capillary channel for sample analysis that is filled with an electrophoresis running buffer. Thereafter, further, the sample that is introduced is subjected to the electrophoresis. The electrophoresis can be performed by creating a potential difference between both ends of the capillary channel for sample analysis with a voltage application unit that will be described below. The capillary channel for sample analysis may be filled with an electrophoresis running buffer in advance or at the time of use.

In the capillary channel for sample analysis, in a case where the cross-sectional shape perpendicular to the capillary channel direction is circular, the diameter thereof is not particularly limited, however is, for example, in the range of 10 to 200 µm, and preferably in the range of 25 to 100 µm. In the capillary channel for sample analysis, in a case where the cross-sectional shape perpendicular to the capillary channel direction is rectangular, for example, the width thereof is in the range of 10 µm to 200 µm and the depth thereof is in the range of 10 µm to 200 µm, and preferably the width thereof is in the range of 25 µm to 100 µm and the depth thereof is in the range of 25 µm to 100 µm. Setting of the capillary channel for sample analysis in the aforementioned range makes it possible to perform further accurate analysis in the capillary electrophoresis analysis apparatus of the present invention. Further, in the present invention, the length of the capillary channel for sample analysis is not particularly limited and is, for example, in the range of 0.5 cm to 15 cm, and preferably in the range of 1 cm to 5 cm.

As described above, the capillary channel may be formed by substrates or may be formed in a substrate by embedding a capillary tube therein. In the former case, the material of the capillary channel is the material of the substrate, for example. In the latter case, the material of the capillary channel is the material of the capillary tube embedded, for example. The material of the capillary channel is not particularly limited, and examples thereof include, as in the above, a glass material such as synthetic silica glass, fused silica, borosilicate glass, etc.; a polymeric material such as polymethylmethacrylate (PMMA), cycloolefin polymer (COP), polycarbonate (PC), polydimethylsiloxane (PDMS), polystyrene (PS), polylactic acid (PLA), polyethylene (PE), polytetrafluoroethylene (PTFE), polyetheretherketone (PEEK), etc; and the like.

The same materials as described above can be employed for the material of the capillary channel for sample analysis. In the case where the channel filled with an electrophoresis running buffer and the channel without an electrophoresis running buffer are irradiated with light having the same light path length and wavelength, the material of the capillary channel for sample analysis is preferably the one that has the following properties: the transmittance in the case of being used for the former channel is 50% or more of the transmittance in the case of being used for the latter channel. Examples of the material showing the foregoing transmittance include, but not limited to, polymer materials such as polystyrene (PS), polyetheretherketone (PEEK), acrylic resins, polycarbonate (PC), polyethylene terephthalate (PET), and the like:

The inner wall of the capillary channel may be applied with a surface treatment, for example, for inhibiting being adsorbed with biologically-derived components in the sample. Examples of the surface treatment include, but not limited to, hydrophilic treatments such as a phosphate treatment, a UV treatment, an alkali treatment, an inorganic nanoparticle coating treatment, a graft copolymerization treatment, a corona discharge treatment, and the like; water-repellent treatments; and the like, and two or more of these treatments may be performed in combination.

Normally, an inner wall of the glass capillary channel is negatively charged. For example, the inner wall of the glass capillary channel may be positively coated, and it is not particularly limited. Further, according to the presence or absence or types of a polar group in polymer, an inner wall of the polymeric capillary channel may be positively or negatively charged or in a condition of charge-free (nonpolar). Although it is not particularly limited, polymer that does not have the polar group may be charged by introducing the polar group, for example. A commercially available capillary tube may be used as the capillary tube, for example.

### < voltage application unit >

As described above, the capillary electrophoresis analysis apparatus of the present invention includes a voltage application unit that contains electrodes. By creating a potential difference between both ends of the capillary channel for sample analysis with the voltage application unit, an electrophoresis of a sample can be performed.

The capillary electrophoresis analysis apparatus of the present invention preferably includes a plurality of electrodes as the voltage application unit. In the capillary electrophoresis analysis apparatus, portions, on which the electrodes are arranged, are not particularly limited. The electrodes may be arranged, for example, in the electrophoresis chip, and specifically, in the liquid reservoirs. In this case, for example, through-holes that are in communication with the liquid reservoirs may be formed in the side surface of the substrate of the electrophoresis chip and electrodes may be inserted into the liquid reservoirs via these through-holes. Alternatively, cyclic electrodes may be used as the electrodes, and arranged on wall surfaces of the liquid reservoirs on which the through-holes are provided. Also, disk-shaped electrodes may be used and arranged on bottom surfaces of the liquid reservoirs.

The material of the electrode is not particularly limited, and examples thereof include stainless steel (SUS), platinum (Pt), and gold (Au), etc.

Although it is not particularly limited, besides the electrode, the voltage application unit may include an electric wire, a voltage generator, etc.

Although it is not particularly limited, for example, as described above, it is preferable that the voltage application unit can adjust voltage to be applied between the electrodes. Since the voltage can suitably be set in accordance with the intended use by such voltage adjustment, an analysis accuracy of the capillary electrophoresis analysis apparatus of the present invention can further be increased. Further, due to such voltage adjustment, the capillary electrophoresis analysis apparatus of the present invention can apply different voltages using the same electrode. Therefore, it is preferable that the voltage application unit further includes a voltage adjustment unit, for example, and an example of the voltage adjustment unit includes a variable voltage generator.

A case where the voltage is applied is not particularly limited, and examples thereof include a case of bubble detection, a case of electrophoresis, and the like. The bubble detection is detection of bubbles generated in the channel when a capillary channel is filled with a solution such as an electrophoresis running buffer, for example. The electrophoresis is an electrophoresis of a sample introduced into the capillary channel for sample analysis, for example. The voltage applied for the bubble detection is not particularly limited, and is, for example, in the range of 0.1 to 1 kV The voltage applied for the electrophoresis is not particularly limited, and is, for example, in the range of 0.5 to 20 kV

### < absorbance measurement unit>

As described above, the capillary electrophoresis analysis apparatus of the present invention includes an absorbance measurement unit for measuring an absorbance of protein in blood of the sample that has been subjected to the electrophoresis.

Although it is not particularly limited, for example, the absorbance of the protein in blood can be calculated from a transmission intensity by irradiating a light of specific wavelength onto a detecting point of the capillary channel for sample analysis and detecting a transmitted light. The absorbance measurement unit is not particularly limited, and may be composed of a light source, an optical filter, a collecting lens, a detection unit, and the like, for example. The light source is not particularly limited, and examples thereof include a light-emitting diode (LED), a semiconductor laser diode (LD), and the like. The optical filter is not particularly limited, and examples thereof include a metallic interference filter, an all-dielectric interference filter, and the like. The collecting lens is not particularly limited, and examples thereof include a double-convex lens, and the like. The detection unit is not particularly limited, and examples thereof include a photodiode, a phototransistor, a photo IC, a photomultiplier tube, and the like.

A spectroscopic method of the absorbance measurement unit is not particularly limited, and examples thereof include a pre-spectroscopic method and a post-spectroscopic method. As described above, the pre-spectroscopic method is preferable. Generally, the pre-spectroscopic method is a method in which light emitted from the light source is dispersed into specific wavelength in advance of irradiation onto a detecting point. In a case of the pre-spectroscopic method, in the capillary electrophoresis analysis apparatus of the present invention, a detecting point of the capillary channel for sample analysis is irradiated with the light of specific wave length.

As described above, in the capillary electrophoresis analysis apparatus of the present invention, the specific wave length, that is a measurement wave length of the absorbance, is, for example, in the range of 260 nm to 300 nm or 380 nm to 450 nm, and preferably in the range of 400 nm to 430 nm.

### < quantitative dispensing unit >

As described above, it is preferable that the capillary electrophoresis analysis apparatus of the present invention further includes a quantitative dispensing unit, for example. Since the quantitative dispensing of a sample, a reagent, and the like, can automatically be performed by means of the quantitative dispensing unit, a simple measurement can be performed.

Although it is not particularly limited, the quantitative dispensing unit may be provided in the electrophoresis chip or may be provided outside of the electrophoresis chip.

In the former case, examples of the quantitative dispensing unit include a measurement channel, and the like. The measurement channel is not particularly limited, however is, for example, a part of the capillary channel of the electrophoresis chip and can pool or retain a certain amount of sample or reagent such as an electrophoresis running buffer or the like. The measurement channel is not particularly limited, and examples thereof include a measurement channel for sample, a measurement channel for electrophoresis running buffer, and the like. Further, in the latter case, the quantitative dispensing unit is not particularly limited, and examples thereof include a quantitative dispensing unit that has a suction and discharge mechanism; and the like.

### < stirring unit >

As described above, it is preferable that the capillary electrophoresis analysis apparatus of the present invention further includes a stirring unit, for example. For example, since a solution such as a sample, a reagent, and the like, can automatically be mixed by means of the stirring unit, a simple measurement can be performed.

The stirring unit is not particularly limited, and examples thereof include a stirring bar, and the like. The stirring bar is not particularly limited, and examples thereof include a small piece of a ferromagnet whose surface is sealed with polytetrafluoroethylene or the like; and the like. A solution or the like in the liquid reservoir can be stirred, for example, by disposing the stirring bar in a mixing liquid reservoir for mixing a sample and a reagent, and providing an electromagnetic stirring machine such as a magnetic stirrer at a bottom surface of the liquid reservoir or the like. Alternatively, for example, the quantitative dispensing unit may serves as the quantitative dispensing unit and the stirring unit. In this case, by means of the quantitative dispensing unit, for example, by sucking and discharging a mixture of a sample and an electrophoresis running buffer, the aforementioned two solutions can be stirred.

### < liquid sending unit >

As described above, it is preferable that the capillary electrophoresis analysis apparatus of the present invention further includes a liquid sending unit for introducing a solution into the capillary channel, for example. Since an electrophoresis chip can automatically be filled or introduced with the solution by means of the liquid sending unit, a simple measurement can be performed. By means of the liquid sending unit, for example, a reagent such as an electrophoresis running buffer, an analytical reagent, a diluent, a washing liquid, or the like; and a sample can be introduced into the capillary channel. The liquid sending unit is not particularly limited, and examples thereof include a suction unit (mechanism), a discharge unit (mechanism), a voltage application unit (mechanism), and the like.

Although it is not particularly limited, for example, the suction unit (pressure reduction unit) may be provided with a pressure reduction pump, a drain portion, and the like. The drain portion may be disposed at one end of the capillary channel, and the pressure reduction pump may be connected to the drain portion, for example. Further, by reducing the pressure in the channel with the pressure reduction pump via the drain, the solution can be sucked and introduced into the capillary channel from the other end of the capillary channel.

Although it is not particularly limited, for example, the discharge unit (pressure unit) may be provided with a pressure pump and a drain portion. The drain portion may be disposed at one end of the capillary channel and the pressure pump may be connected to the drain portion, for example. Further, by applying pressure to an inside of the channel by discharging air thereinto with the pressure pump via the drain, the solution can be introduced into the capillary channel by discharging air from the one end of the capillary channel.

Alternatively, the voltage application unit may be composed of, for example, an electrode, an electric wire, a power source, and the like, or may be the aforementioned voltage application unit. According to the voltage application unit, a solution such as a sample or the like can be introduced into the capillary channel by applying voltage on both ends of a capillary channel and using an electroosmotic flow thus generated.

### < stray light removing unit >

As described above, it is preferable that the capillary electrophoresis analysis apparatus of the present invention further includes a stray light removing unit, for example. For example, since a measurement accuracy of absorbance is further improved by including the stray light removing unit, a highly accurate measurement can be performed.

In the present invention, the stray light means a light that is not contributing to detection of the transmitted light. The stray light removing unit is not particularly limited, and examples thereof include an aperture, a slit, a pinhole, and the like, which are arranged between the light source and the capillary channel for sample analysis. The shape of a hole of the aperture, the slit, and the pinhole is not particularly limited, and examples thereof include circular, rectangular, and the like. In a case where the shape of the hole of the aperture, the slit, and the pinhole is circular, the diameter thereof is not particularly limited, however may be in the same range as an inner diameter of the capillary channel, for example. In a case where the shape of the hole of the aperture, the slit, and the pinhole is rectangular, the length in the short side direction of the hole is not particularly limited, however may be in the same range as an inner diameter of the capillary channel, for example.

### < position adjustment unit >

As described above, it is preferable that the capillary electrophoresis analysis apparatus of the present invention further includes a position adjustment unit, for example. In the capillary electrophoresis analysis apparatus of the present invention, the position adjustment unit is, for example, a unit for adjusting at least one of a position of the capillary channel for sample analysis and a position of a light flux so that the light flux of specific wavelength can accurately be entered at a detecting point of the capillary channel for sample analysis. Since the position of at least one of the light flux and the capillary channel for sample analysis can be adjusted by means of the position adjustment unit, an analysis accuracy of the capillary electrophoresis analysis apparatus of the present invention can further be improved.

Although it is not particularly limited, for example, the position adjustment unit can adjust the aforementioned position in at least one of the direction of the diameter of the capillary channel for sample analysis and the direction parallel to the channel, and preferably, the position adjustment unit can adjust the position in both of the direction of the diameter of the capillary channel and the direction parallel to the channel. The position adjustment unit is not particularly limited, and examples thereof include a light source transfer unit (mechanism), an electrophoresis chip transfer unit (mechanism), and the like.

A method of adjusting a position by the light source transfer unit (mechanism) is, for example, as follows. First, the capillary channel for sample, analysis is irradiated with a light flux while transferring a light source or the like in the direction of the diameter of the capillary channel for sample analysis or the direction parallel to the channel by means of the light source transfer unit (mechanism), and a light scattered by the capillary channel for sample analysis is detected. When a wall of the capillary channel for sample analysis is irradiated with the light flux, the scattered light is detected as a peak value. In this state, by positioning the light source or the like at an intermediate position between two peaks, it can be adjusted that the light flux enters the detecting point at a center portion of the capillary channel for sample analysis.

The electrophoresis chip transfer unit (mechanism) can detect the scattered light and adjust an incident light on the capillary channel for sample analysis in the same manner as the light source transfer unit (mechanism) except that the electrophoresis chip is transferred instead of the light source or the like. In the capillary electrophoresis analysis apparatus of the present invention, as the position adjustment unit, one of the transfer unit (mechanisms) may be provided or two or more of the transfer unit (mechanisms) may be provided.

### < contaminant removal unit>

Preferably, the capillary electrophoresis analysis apparatus of the present invention further comprises, for example, a contaminant removal unit as described above. In the capillary electrophoresis analysis apparatus of the present invention, the contaminant removal unit is a unit that can remove contaminants in the sample, for example. The contaminant removal unit may be arranged in a microchip between the capillary channel for sample analysis and the liquid reservoir in which the sample is introduced or stored or arranged as a mechanism that is formed outside of the microchip between the microchip and a mechanism in which the sample is introduced or stored. By arranging the contaminant removal unit in this manner, for example, contaminants contained in the sample can be removed before the sample is introduced into the capillary channel for sample analysis. As a result, for example, the decline in analysis accuracy due to the contaminants can be inhibited. Examples of the contaminant removal unit include, but not limited to, pre-filters, and the like. Examples of the filter material used for the pre-filter include, but not limited to, metals, polyether ether ketone (PEEK), polyethylene (PE), acrylic resins, nylon®, cottons, wool, and the like. The material may be applied with a surface treatment, for example, for inhibiting being adsorbed with the protein in blood or the like. The pre-filter may comprise one or more of the filters of the aforementioned materials. Examples of the contaminants include, but not limited to, cell membranes, proteins, and lipid in a sample of blood which is subjected to a hemolysis treatment.

### <air vent unit>

Preferably, the capillary electrophoresis analysis apparatus of the present invention further comprises an air vent unit as described above. In the capillary electrophoresis analysis apparatus of the present invention, the air vent unit is a unit for removing air (air bubble) from the capillary channel, for example. Preferably, the air vent unit is arranged between the liquid reservoir in which the sample is introduced or stored and the detecting point on the capillary channel for sample analysis irradiated with light of a specific wavelength for the absorbance measurement as described above. By arranging the air vent unit in this manner, for example, air in the capillary channel for sample analysis can be removed. As a result, for example, the decline in analysis accuracy due to the air (air bubble) can be inhibited. Examples of the air vent unit include, but not limited to, structures capable of opening and closing a channel for removing the air (air bubble). The structures capable of opening and closing are not particularly limited, and for example, conventionally known structures such as a valve structure, a channel in channel structure, and the like can be employed.

### < electrophoresis running buffer>

As described above, it is preferable that the capillary electrophoresis analysis apparatus of the present invention further includes an electrophoresis running buffer, for example. The electrophoresis running buffer may be provided in a liquid reservoir, a capillary channel, or the like in an electrophoresis chip, or may be provided outside of the electrophoresis chip. The electrophoresis running buffer is not particularly limited, and examples thereof include a buffer solution, and the like. The buffer solution is not particularly limited, and examples thereof include morpholinoethanesulfonic acid (MES), N-(2-acetamido)iminodiacetic acid (ADA), N-(2-acetamido)-2-aminoethanesulfonic acid (ACES), N,N-bis(2-hydroxyethyl)-2-aminoethanesulfonic acid (BES), 3-morpholinopropanesulfonic acid (MOPS), N-tris(hydroxymethyl)methyl-2-aminoethanesulfonic acid (TES), 2-[4-(2-hydroxyethyl)-1-piperazinyl]ethanesulfonic acid (HEPES), etc. The buffer solution may contain acid or weak base, although it is not particularly limited. The acid is not particularly limited, and examples thereof include maleic acid, tartaric acid, succinic acid, fumaric acid, phthalic acid, malonic acid, malic acid, etc. The weak base is not particularly limited, and examples thereof include arginine, lysine, histidine, tris, etc.

### < diluent >

As described above, it is preferable that the capillary electrophoresis analysis apparatus of the present invention further includes a diluent for diluting a sample, for example. The diluent may be provided in a liquid reservoir or the like in the electrophoresis chip, or may be provided outside of the electrophoresis chip. The diluent is not particularly limited, and examples thereof include distilled water, the aforementioned buffer solution, etc., and buffer solution is preferable.

### <sample>

In the present invention, examples of the sample that contains protein in blood include a blood sample, a sample prepared by subjecting the blood sample to a hemolysis treatment, and the like. The blood sample is not particularly limited, and examples thereof include whole blood, blood plasma, blood serum, and the like, and the whole blood is preferable. Examples of the hemolysis treatment include a surfactant treatment, an osmotic pressure treatment, a sonication treatment, a freeze/thaw treatment, a pressure treatment, etc., and the surfactant treatment, the osmotic pressure treatment, and the sonication treatment are preferable.

The surfactant treatment is not particularly limited and, for example, it may be a treatment in which the blood sample is hemolyzed with a diluent to which a surfactant is added. Examples of the surfactant include saponin, polyoxyethylene surfactant ("Triton X-100" (trade name) manufactured by Nacalai Tesque, Inc.), etc. The diluent is not particularly limited, and examples thereof include distilled water, the aforementioned buffer solution, and the like. The osmotic pressure treatment is not particularly limited, it may be a treatment in which the blood sample is hemolyzed with a solution that is adjusted to have low osmotic pressure. The solution is not particularly limited, and examples thereof include distilled water, a diluent that is adjusted to have low osmotic pressure, and the like, and the distilled water is preferable. The diluent is not particularly limited, and examples thereof include the aforementioned buffer solution, and the like. The sonication treatment is not particularly limited, and a publicly known ultrasonic processor can be used. The processing conditions thereof are not particularly limited.

In the present invention, the protein in blood is protein contained in blood. The protein in blood is not particularly limited, and examples thereof include hemoglobin (Hb), albumin (Alb), globulin (α1, α2, β, r globulin), fibrinogen, C-reactive protein (CRP), rheumatoid factor (RF), glutamic oxalacetic transaminase (GOT), glutamic pyruvic transaminase (GPT), creatine phosphokinase (CPK), amylase (Amy), r-glutamyl transferase (GGT), alkaline phosphatase (ALP), fructosamine (FRA), anti-streptolysin O (ASO), etc., and hemoglobin is preferable.

The hemoglobin is not particularly limited and examples thereof include normal hemoglobin (HbA0), glycated hemoglobin, modified hemoglobin, genetic variant hemoglobin, fetal hemoglobin (HbF), etc. Examples of the glycated hemoglobin include hemoglobin A1a (HbA1a), hemoglobin Alb (HbA1b), hemoglobin A1c (HbA1c), GHbLys, etc. Examples of the hemoglobin A1c include stable HbAlc, unstable HbA1c, etc. Examples of the modified hemoglobin include carbamoylated Hb, acetylated Hb, etc. Examples of the variant hemoglobin include hemoglobin S (HbS), hemoglobin C (HbC), hemoglobin M (HbM), hemoglobin H (HbH), etc. As described above, the hemoglobin is preferably hemoglobin A1c (HbA1c), hemoglobin F (HbF), hemoglobin A2 (HbA2), hemoglobin S (HbS), and hemoglobin C (HbC), and more preferably hemoglobin A1c (HbA1c).

In the capillary electrophoresis analysis apparatus of the present invention, although it is not particularly limited, among the aforementioned protein in blood, the hemoglobin is preferable as a measurement item, and hemoglobin A1c (HbAlc) is more preferable. The measurement item may be one item or a combination of two or more items, for example, and it is not particularly limited.

The capillary electrophoresis analysis apparatus of the present invention may further calculate a concentration or a ratio of protein in blood on the basis of an absorbance of the measured protein in blood, and an analysis item may be the concentration or the ratio of the protein in blood, for example. The ratio or the concentration is not particularly limited, and examples thereof include a hemoglobin concentration, an albumin concentration, a globulin concentration, a ratio of various hemoglobin, an albumin/glycated albumin ratio, an albumin/globulin ratio, an albumin/creatinine ratio, etc., and the hemoglobin concentration and the hemoglobin ratio are preferable. The hemoglobin concentration is not particularly limited, and examples thereof include a concentration of the aforementioned various hemoglobin, and a hemoglobin A1c concentration, a hemoglobin F concentration, a hemoglobin A2 concentration, a hemoglobin S concentration, and a hemoglobin C concentration are preferable, and the hemoglobin A1c concentration is more preferable. The hemoglobin ratio is not particularly limited, and examples thereof include a ratio of the aforementioned various hemoglobin, and a hemoglobin A1c ratio, a hemoglobin F ratio, a hemoglobin A2 ratio, a hemoglobin S ratio, and a hemoglobin C ratio are preferable, and a hemoglobin A1c ratio is more preferable.

### [Examples]

Next, the capillary electrophoresis analysis apparatus of the present invention is explained with Examples. However, the present invention is not limited to the following Examples.

### [Example 1]

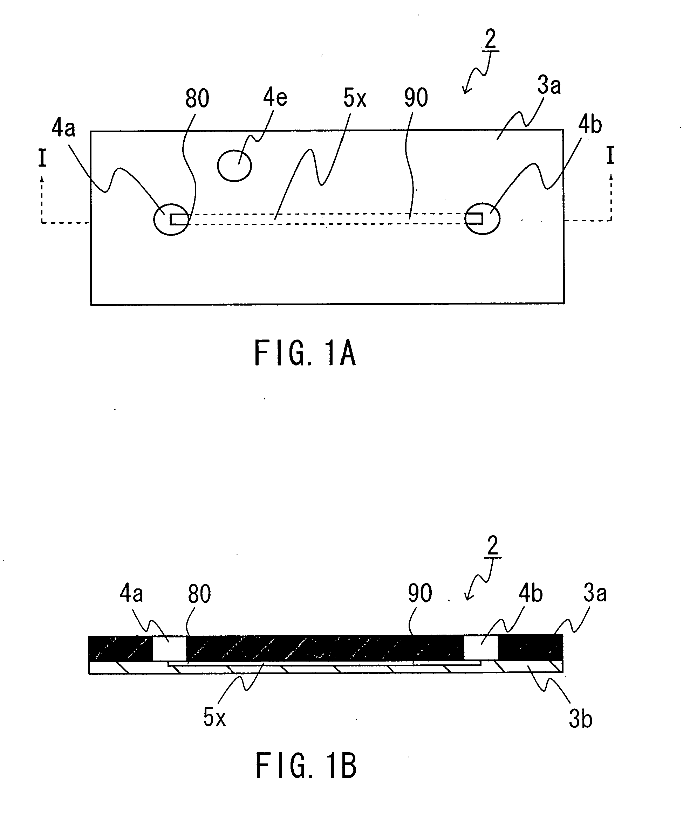

An electrophoresis chip used for the capillary electrophoresis analysis apparatus of this Example is shown in FIG. 1. FIG. 1A is a plane view of the electrophoresis chip. FIG. 1B is a cross-sectional view viewed in the direction of line I-I of the electrophoresis chip shown in FIG. 1A. FIG. 1 is schematic diagrams for easier understanding, and the size, proportions and like features of each component are not limited thereto and may be different therefrom. As shown in FIG. 1, the electrophoresis chip 2 includes a lower substrate 3b and an upper substrate 3a, and the upper substrate 3a is laminated onto the lower substrate 3b. Three through-holes are formed in the upper substrate 3a. The bottom parts of the three through-holes of the upper substrate 3a are sealed with the lower substrate 3b by laminating the upper substrate 3a onto the lower substrate 3b, and thereby three concave portions are formed in the electrophoresis chip 2. They serve as liquid reservoirs 4a, 4b, and 4e, respectively. A groove having a shape of "I" is formed on the lower substrate 3b. The upper part of the groove having a shape of "I" of the lower substrate 3b is sealed with the upper substrate 3a by laminating the upper substrate 3a onto the lower substrate 3b in such a manner that a forming face of this groove faces to the upper substrate 3a, and thereby a channel is formed in the electrophoresis chip 2. This channel serves as a capillary channel for sample analysis 5x. The liquid reservoir 4a and the liquid reservoir 4b are in communication with each other via the capillary channel for sample analysis 5x. In contrast, the liquid reservoir 4e is not in communication with the capillary channel for sample analysis 5x and is provided as an independent liquid reservoir. An end of the capillary channel for sample analysis 5x at the liquid reservoir 4a side serves as an electrophoresis starting point 80. Further, a point on the capillary channel for sample analysis 5x between the liquid reservoir 4a and the liquid reservoir 4b serves as a detecting point 90.

The electrophoresis chip 2 is rectangular parallelepiped. However, the present invention is not limited thereto. In the capillary electrophoresis analysis apparatus of the present invention, the electrophoresis chip 2 may be in any form as long as it does not adversely affect the analysis of the sample which will be described below. Furthermore, the electrophoresis chip 2 is composed of two substrate pieces (an upper substrate 3a and a lower substrate 3b). However, the present invention is not limited thereto. In the capillary electrophoresis analysis apparatus of the present invention, the electrophoresis chip may be composed of a single-piece substrate.

In the electrophoresis chip 2, the length and the width of the upper substrate 3a correspond to the maximum length and the maximum width of the whole electrophoresis chip described above. Therefore, the length and the width of the upper substrate 3a are arranged to be identical to the maximum length and the maximum width of the whole electrophoresis chip described above, respectively. In the electrophoresis chip 2, the thickness of the upper substrate 3a can be designed suitably according to the volume of the liquid reservoirs 4a, 4b, and 4e, however is, for example in the range of 0.1 mm to 3 mm, and preferably in the range of 1 mm to 2mm.

In the electrophoresis chip 2, for example, the length and the width of the lower substrate 3b are the same as the length and the width of the upper substrate 3a, respectively. The thickness of the lower substrate 3b is not particularly limited, however is, for example, in the range of 0.1 mm to 3 mm, and preferably in the range of 0.1 mm to 1 mm.

The material of the upper substrate 3a and the lower substrate 3b is not particularly limited as long as it does not adversely affect the measurement of the absorbance. Examples of the material of the upper substrate 3a and the lower substrate 3b include the aforementioned materials.

The width and the depth of the capillary channel for sample analysis 5x is not particularly limited, however for example, the width is in the range of 25 µm to 100 µm and the depth is in the range of 25 µm to 100 µm.

The volume of the liquid reservoirs 4a, 4b, and 4e is as described above. In FIG. 1, the form of the liquid reservoirs 4a, 4b, and 4e is cylindrical. However, the present invention is not limited thereto. In the present invention, the form of the liquid reservoirs 4a, 4b, and 4e may be an arbitrary form as described above.

The maximum thickness of the electrophoresis chip 2 is the sum of the thickness of the upper substrate 3a and the lower substrate 3b. Respective thickness of the upper substrate 3a and the lower substrate 3b are as described above.

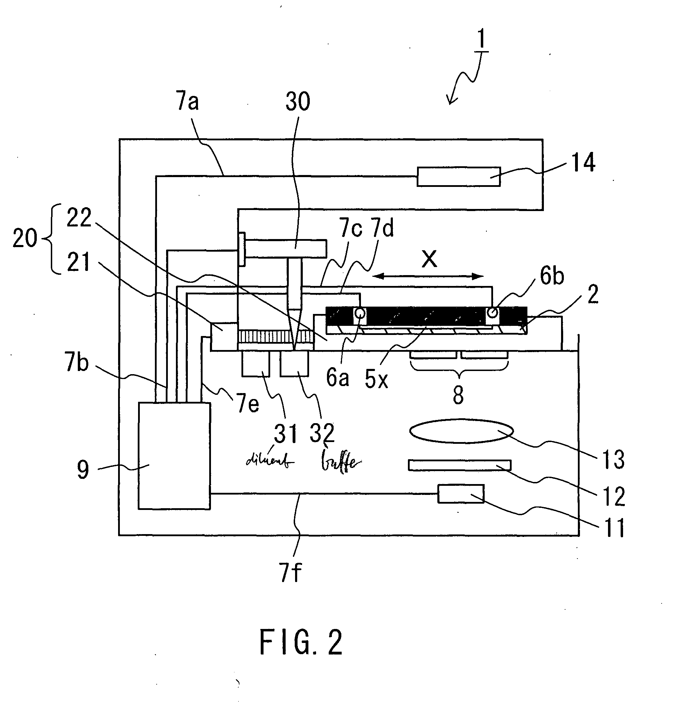

A capillary electrophoresis analysis apparatus of this Example is shown in FIG. 2. FIG. 2 is a schematic diagram for easier understanding, and the size, proportions and like features of each component are not limited thereto and may be different therefrom. As shown in FIG. 2, this capillary electrophoresis analysis apparatus 1 includes the aforementioned electrophoresis chip 2, electrodes 6a and 6b, electric wires 7a to 7f, a slit 8, a control unit 9, a light source 11, an optical filter 12, a collecting lens 13, a detection unit 14, an electrophoresis chip transfer unit (mechanism) 20, a quantitative dispensing unit 30, a diluent 31, and an electrophoresis running buffer 32. The electrophoresis chip transfer unit (mechanism) 20 contains a drive unit 21 and a stage 22. The electrophoresis chip 2 is arranged on the stage 22. The electrodes 6a and 6b are arranged in the liquid reservoirs 4a and 4b of the electrophoresis chip 2, respectively. The detection unit 14, the quantitative dispensing unit 30, the electrodes 6a and 6b, the electrophoresis chip transfer unit (mechanism) 20, and the light source 11 are connected to the control unit 9 via the electric wires 7a to 7f, respectively. The control unit 9 controls power supply or the like to the aforementioned components which are connected thereto via the electric wires 7a to 7f.

In the capillary electrophoresis analysis apparatus 1, the stage 22 is movable in a horizontal biaxial direction (an X-Y direction) by the drive unit 21 that is connected to an end thereof. The X direction and the Y direction vertically intersect on the horizontal surface. Thereby, the position of the electrophoresis chip 2 can be adjusted. Since the position of the electrophoresis chip 2 is adjusted by the electrophoresis chip transfer unit (mechanism) 20, the detecting point 90 can accurately be irradiated with the light flux of specific wavelength. Further, the quantitative dispensing unit 30 can perform a quantitative analysis of the diluent 31 and the electrophoresis running buffer 32, respectively, and can dispense them to the liquid reservoir 4a or the liquid reservoir 4e of the electrophoresis chip 2. By applying the voltage between the electrodes 6a and 6b, an electrophoresis of a sample that is introduced into the capillary channel for sample analysis 5x can be performed. Then, the light emitted from the light source 11 is dispersed into specific wavelength by the optical filter 12 and converged by the collecting lens 13, as well as the amount of light is increased and the stray light is removed by the slit 8, and then the sample at the detecting point 90 on the capillary channel for sample analysis 5x of the electrophoresis chip 2 is irradiated. The transmitted light of the light irradiated on the detecting point 90 is detected by the detection unit 14. Measurement of an absorbance of thus detected transmitted light makes it possible to analyze protein in blood to be analyzed contained in the sample.

The method of manufacturing the electrophoresis chip 2 of the capillary electrophoresis analysis apparatus 1 of this Example is not particularly limited and conventionally known methods can suitably be applied.

Next, the method of analyzing the protein in blood using the capillary electrophoresis analysis apparatus 1 of this Example is explained.

First, the electrophoresis running buffer 32 is prepared. The electrophoresis running buffer 32 is not particularly limited, however is, for example, a solution prepared by adding chondroitin C in a proportion of 0.8 wt% to a solution of 100 mmol/L containing fumaric acid and arginine acid, the solution being adjusted to pH 4.8. Next, the electrophoresis chip 2 is attached to the stage 22 and disposed in the capillary electrophoresis analysis apparatus 1. Then, a certain amount of the electrophoresis running buffer 32 is injected into the liquid reservoir 4a by the quantitative dispensing unit 30. Further, the pressure in the capillary channel for sample analysis 5x is reduced by connecting a pressure reduction pump (not shown) to the liquid reservoir 4b and the capillary channel for sample analysis 5x is filled with the electrophoresis running buffer 32.

Next, the diluent 31 is injected into the liquid reservoir 4e by the quantitative dispensing unit 30. Further, a human whole blood is added to the reservoir 4e as a sample and is stirred by pipetting, and thus a mixture of the sample and the diluent 31 is prepared. As the diluent 31, distilled water or the like can be used. Subsequently, the mixture is injected into the liquid reservoir 4a. Then, the voltage is applied to the electrodes 6a and 6b, which are respectively arranged in the liquid reservoirs 4a and 4b, thereby creating a potential difference between both ends of the capillary channel for sample analysis 5x. The sample is thereby moved from the electrophoresis starting point 80 to the liquid reservoir 4b side. The voltage is not particularly limited, however is, for example, in the range of 0.5 to 20kV.

Next, in the same manner as described above, the light is dispersed and collected, and then the detecting point 90 is irradiated with the light (wave length of 415 nm), from which a stray light is further removed. Then, the transmitted light at the detecting point 90 is detected by the detection unit 14 and the absorbance of the protein in blood in the sample is measured. Electropherogram is made that indicates relationship between degree of the obtained absorbance and analysis time (from start of electrophoresis to detection).

### [Example 2]

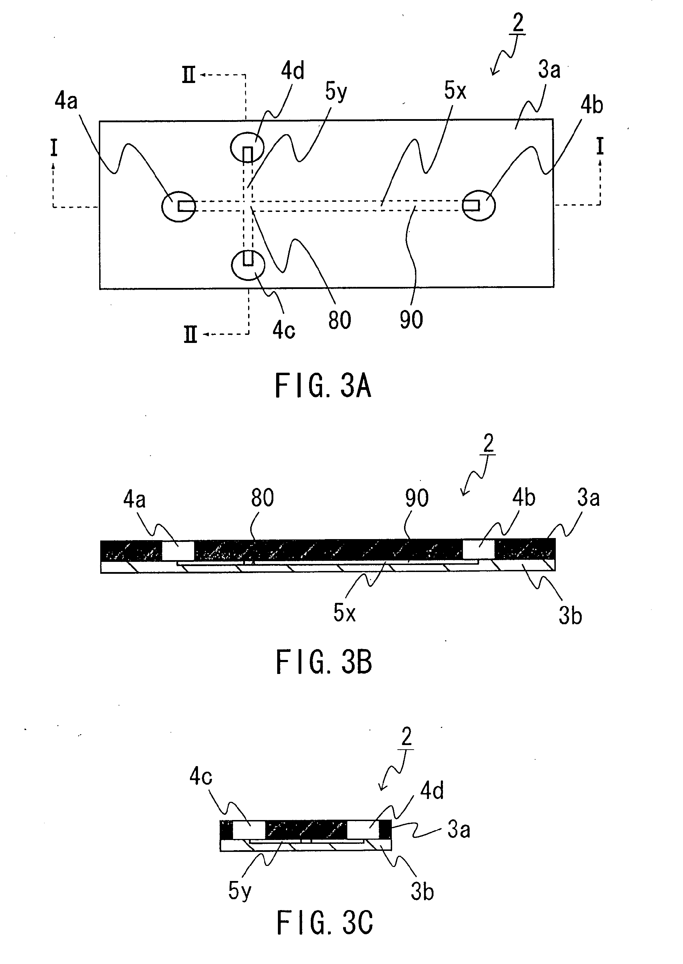

An electrophoresis chip used for the capillary electrophoresis analysis apparatus of this Example is shown in FIG. 3. In FIG. 3, the portions that are identical to those in FIG. 1 are given the same numbers and symbols. FIG. 3A is a plane view of the electrophoresis chip, FIG. 3B is a cross-sectional view viewed in the direction of line I-I of the electrophoresis chip shown in FIG. 3A, and FIG. 3C is a cross-sectional view viewed in the direction of line II-II of the electrophoresis chip shown in FIG. 3A. FIG.3 is schematic diagrams for easier understanding, and the size, proportions and like features of each component are not limited thereto and may be different therefrom.

As shown in FIG. 3, the electrophoresis chip 2 is composed of a lower substrate 3b and an upper substrate 3a, the upper substrate 3a being laminated onto the lower substrate 3b. Plural through-holes (four in this Example) are formed in the upper substrate 3a. The bottom parts of the four through-holes formed in the upper substrate 3a are sealed with the lower substrate 3b and, thereby four liquid reservoirs 4a to 4d are formed. A cross-shaped groove is formed on the lower substrate 3b. By sealing the upper part of the cross-shaped groove formed on the lower substrate 3b with the upper substrate 3a, a capillary channel for sample analysis 5x and a capillary channel for sample introduction 5y are formed. The liquid reservoir 4a and the liquid reservoir 4b are in communication with each other via the capillary channel for sample analysis 5x. The liquid reservoir 4c and the liquid reservoir 4d are in communication with each other via the capillary channel for sample introduction 5y. The capillary channel for sample analysis 5x and the capillary channel for sample introduction 5y intersect. The capillary channel for sample analysis 5x and the capillary channel for sample introduction 5y are in communication with each other at the intersection. The intersection serves as an electrophoresis starting point 80. Further, a point on the capillary channel for sample analysis 5x between the liquid reservoir 4a and the liquid reservoir 4b serves as a detecting point 90.

In the electrophoresis chip 2, the maximum length of the capillary channel for sample analysis 5x is different from that of the capillary channel for sample introduction 5y. However, the present invention is not limited thereto. In the capillary electrophoresis analysis apparatus of the present invention, the maximum length of the capillary channel for sample analysis 5x may be the same as the maximum length of the capillary channel for sample introduction 5y.

The electrophoresis chip 2 has the same configuration as the electrophoresis chip shown in FIG. 1 except that the liquid reservoirs 4c and 4d and the capillary channel for sample introduction 5y are formed as well as the liquid reservoir 4e is not formed. The width and the depth of the capillary channel for sample introduction 5y are the same as the width and the depth of the capillary channel for sample analysis 5x. The volume and the form of the liquid reservoirs 4c and 4d are the same as those of the electrophoresis chip shown in FIG. 1.

A capillary electrophoresis analysis apparatus 1 of this Example has the same configuration as the capillary electrophoresis analysis apparatus shown in FIG. 2 except that the electrophoresis chip 2 is the electrophoresis chip shown in FIG. 3 instead of the electrophoresis chip shown in FIG. 1 as well as electrodes 6c and 6d (not shown) are arranged in the liquid reservoirs 4c and 4d of the electrophoresis chip 2.

Next, the method of analyzing the protein in blood using the capillary electrophoresis analysis apparatus 1 of this Example is explained.

First, the electrophoresis chip 2 is attached to a stage 22 and disposed in the capillary electrophoresis analysis apparatus 1. Subsequently, in the same manner as in Example 1, the electrophoresis running buffer 32 is injected into the liquid reservoir 4a by the quantitative dispensing unit 30. Next, in the same manner as in Example 1, the pressure in the capillary channel for sample analysis 5x is reduced by connecting a pressure reduction pump (not shown) to the liquid reservoir 4b, and the capillary channel for sample analysis 5x is filled with the electrophoresis running buffer 32 by the quantitative dispensing unit 30. Then, the electrophoresis running buffer 32 is injected into the liquid reservoir 4c. Further, the pressure in the capillary channel for sample introduction 5y is reduced by connecting a pressure reduction pump (not shown) to the liquid reservoir 4d, and the capillary channel for sample introduction 5y is filled with the electrophoresis running buffer 32.

Next, the diluent 31 is injected into the liquid reservoir 4c by the quantitative dispensing unit 30. Further, a human whole blood is added thereto as a sample and is stirred by pipetting. Then, the voltage is applied to the electrodes 6c and 6d, thereby creating a potential difference between both ends of the capillary channel for sample introduction 5y. The sample is thereby moved to the intersection of the capillary channel for sample analysis 5x and the capillary channel for sample introduction 5y. The voltage applied between the electrodes 6c and 6d is not particularly limited, however is, for example, in the range of 0.5 to 20kV

Next, the voltage is applied to the electrodes 6a and 6b, thereby creating a potential difference between both ends of the capillary channel for sample analysis 5x. The sample is thereby moved from the electrophoresis starting point 80 to the liquid reservoir 4b side. The voltage is not particularly limited, however is, for example, in the range of 0.5 to 20kV.

Next, in the same manner as in Example 1, the light is dispersed and collected, and then the detecting point 90 is irradiated with the light (wave length of 415 nm), from which a stray light is further removed. Then, the transmitted light at the detecting point 90 is detected by the detection unit 14 and the absorbance of the protein in blood in the sample is measured. Electropherogram is made that indicates relationship between degree of the obtained absorbance and electrophoresis time.

### [Example 3]

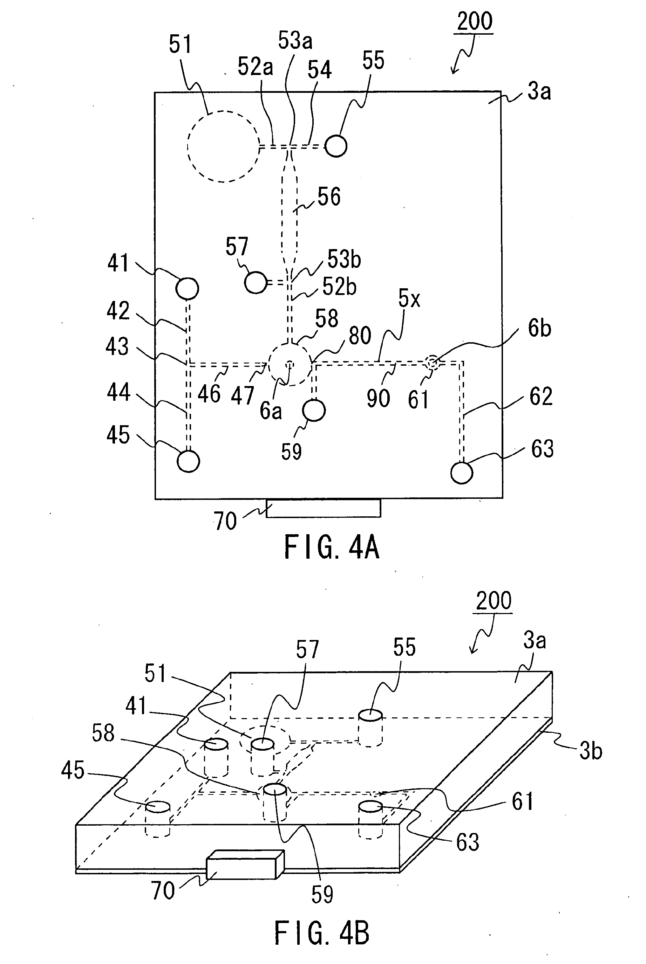

An electrophoresis chip used for the capillary electrophoresis analysis apparatus of this Example is shown in FIG. 4. In FIG. 4, the portions that are identical to those in FIG. 1 and FIG. 3 are given the same numbers and symbols. FIG.4A is a plane view of the electrophoresis chip, and FIG. 4B is a perspective view of the electrophoresis chip. FIG. 4 is schematic diagrams for easier understanding, and the size, proportions and like features of each component are not limited thereto and may be different therefrom. As shown in FIG. 4, the electrophoresis chip 200 includes a lower substrate 3b, an upper substrate 3a, and a connector 70. The connector 70 is arranged on a side surface of a laminated body in which the upper substrate 3a is laminated onto the lower substrate 3b. A wiring pattern (not shown) is formed on the lower substrate 3b.

Six through-holes are formed in the upper substrate 3a. The bottom parts of the six through-holes are sealed with the lower substrate 3b, and thereby six liquid reservoirs are formed. The six liquid reservoirs serve as a sample introduction portion (a sample reservoir) 41, a drain 45, a drain 55, a drain 57, a drain 59, and a drain 63, respectively. Further, three concave portions of various sizes are formed at the bottom surface of the upper substrate 3a. Openings of two concave portions out of the three concave portions are sealed with the lower substrate 3b, and thereby two liquid reservoirs are formed. The two liquid reservoirs serve as a reagent reservoir 51 and a diluent reservoir 58, respectively. An electrophoresis running buffer is sealed in the reagent reservoir 51. An electrode 6a connected to a wiring of the wiring pattern is arranged in the diluent reservoir 58, and a stirring bar (not shown) is sealed in the diluent reservoir 58. An opening of the other one of the concave portion out of the three concave portions is sealed with the lower substrate 3b, and thereby an electrode arrangement portion 61 is formed. An electrode 6b connected to a wiring of the wiring pattern is arranged in the electrode arrangement portion 61. Further, plural grooves are formed on the bottom surface of the upper substrate 3a. Openings of the plural grooves are sealed with the lower substrate 3b, and thereby channels are formed, through which the six reservoirs and the three concave portions are in communication with one another. A capillary channel, through which the diluent reservoir 58 and the electrode arrangement portion 61 are in communication with each other, serves as the capillary channel for sample analysis 5x. An end portion of the capillary channel for sample analysis 5x at the diluent reservoir 58 side serves as an electrophoresis starting point 80. Further, a point on the capillary channel for sample analysis 5x serves as a detecting point 90. Details of channels other than the capillary channel for sample analysis 5x will be described later.

The sample introduction portion 41 is in communication with the drain 45 via a sample introduction channel 42, a branching portion43, and an overflow channel 44 in order. Further, the sample introduction portion 41 is also in communication with the diluent reservoir 58 from the branching portion43 via a sample measurement channel 46. An opening of the sample introduction portion 41 is a sample introduction opening for introducing a sample, which contains protein in blood to be analyzed, into an electrophoresis chip. At an end portion of the sample measurement channel 46 at the diluent reservoir 58 side, an orifice 47 having a narrow channel cross-sectional area is formed.

An electrophoresis chip 200 can measure and introduce the sample, for example, as follows. First, after introducing a sample into the sample introduction portion 41, the sample is sucked from the sample introduction portion 41 by reducing the pressure in a channel that is in communication with the drain 45 with a pressure reduction pump or the like (not shown) that is connected to the drain 45. Due to the suction, a sample that exceeds the volume of the sample measurement channel 46 between the branching portion43 and the orifice 47 flows into the overflow channel 44. Subsequently, the drain 45 is closed and an air is discharged with a pressure pump (not shown) that is connected to the sample introduction portion 41, and the pressure is applied to an inside of a channel that is connected to the sample introduction portion 41. Thereby, a sample corresponding to the volume of the sample measurement channel 46 is introduced into the diluent reservoir 58. Therefore, the introduction amount is set at the volume of the sample measurement channel 46, for example, and accordingly a measurement introduction can be performed.

The reagent reservoir 51 is in communication with the drain 55 via a reagent introduction channel 52a, a branching portion 53a, and an overflow channel 54 in order. Further, the reagent reservoir 51 is also in communication with the diluent reservoir 58 from the branching portion 53a via a reagent measurement channel 56, a branching portion 53b, and a reagent introduction channel 52b. At an end portion of a channel that is branched at the branching portion 53b, the drain 57 is formed. Further, at an end portion of a channel that is branched at an end portion of the capillary channel for sample analysis 5x at the diluent reservoir 58 side, a drain 59 is formed. Furthermore, between the electrode arrangement portion 61 and the drain 63, a flow amount measurement channel 62 is formed.

For example, the electrophoresis chip 200 can make the capillary channel for sample analysis 5x being filled with the electrophoresis running buffer, and introduce the electrophoresis running buffer into the diluent reservoir 58 by measuring it as follows. First, the sample introduction portion 41, the drains 45, 55, 57, and 59 are closed, an air is sucked with a pressure reduction pump or the like (not shown) that is connected to the drain 63, and thereby reducing the pressure in channels and liquid reservoirs which are in communication with the drain 63. Thereby, the reagent introduction channels 52a and 52b, the reagent measurement channel 56, the diluent reservoir 58, the capillary channel for sample analysis 5x, the electrode arrangement portion 61, and the flow amount measurement channel 62 are filled with an electrophoresis running buffer which is sealed in the reagent reservoir 51. Subsequently, the reagent reservoir 51 is closed, the drain 59 is opened, and an air is sucked with a pressure reduction pump or the like (not shown) that is connected to the drain 57, and the pressure is reduced in channels and liquid reservoirs which are in communication with the drain 57. Thereby, an electrophoresis running buffer in the reagent introduction channel 52b and the diluent reservoir 58 is removed. Further, the drain 57 is closed, the drain 55 is opened, and an air is sucked with a pressure reduction pump or the like (not shown) that is connected to the drain 59, and the pressure is reduced in channels and liquid reservoirs which are in communication with the drain 59. Thereby, an electrophoresis running buffer corresponding to the volume of the reagent measurement channel 56 is introduced into the diluent reservoir 58. Therefore, the introduction amount is set at the volume of the reagent measurement channel 56, for example, and accordingly a measurement introduction can be performed. Further, as described above, the sample is introduced into the diluent reservoir 58. Then, the sample and the electrophoresis running buffer can be mixed by rotating the stirring bar (not shown) in the diluent reservoir 58 by a magnetic stirrer (not shown). In this state, in this Example, a surfactant may be added to the electrophoresis running buffer for enabling a hemolysis treatment.

In the electrophoresis chip 200, the length and the width of the upper substrate 3a are, for example, in the range of 10 mm to 200 mm, and preferably in the range of 20 mm to 100 mm. Further, the thickness of the upper substrate 3a is, for example, in the range of 0.1 mm to 10 mm, and preferably in the range of 1 mm to 5 mm.

In the electrophoresis chip 200, the length and the width of the lower substrate 3b are the same as that of the upper substrate 3a. The thickness of the lower substrate 3b is, for example, in the range of 0.1 mm to 10 mm.

In the electrophoresis chip 200, the material of the upper substrate 3a and the lower substrate 3b is not particularly limited as long as it does not adversely affect the measurement of the absorbance. For example, the aforementioned materials can be used as the material of the upper substrate 3a and the lower substrate 3b. Further, the lower substrate 3b is composed by laminating plural substrates formed of the aforementioned materials. Between the plural substrates, wiring patterns made of copper foil or the like are formed.

In the electrophoresis chip 200, with respect to the diameter and the depth of the sample introduction portion 41, for example, the diameter is in the range of 0.1 mm to 10 mm and the depth is in the range of 0.1 mm to 10 mm, and preferably, the diameter is in the range of 1 mm to 5mm and the depth is in the range of 1 mm to 5 mm.

In the electrophoresis chip 200, with respect to the diameter and the depth of the reagent reservoir 51, for example, the diameter is in the range of 0.5 mm to 50 mm and the depth is in the range of 0.1 mm to 10 mm, and preferably, the diameter is in the range of 1 mm to 20 mm and the depth is in the range of 1 mm to 5 mm.

In the electrophoresis chip 200, with respect to the diameter and the depth of the diluent reservoir 58, for example, the diameter is in the range of 0.5 mm to 50mm and the depth is in the range of 0.1 mm to 10 mm, and preferably, the diameter is in the range of 1 mm to 10 mm and the depth is in the range of 1 mm to 5 mm.

In the electrophoresis chip 200, with respect to the diameter and the depth of the drains 45, 55, 57, 59, and 63, for example, the diameter is in the range of 0.1 mm to 10 mm and the depth is in the range of 0.1 mm to 10 mm respectively, and preferably, the diameter is in the range of 1 mm to 5 mm and the depth is in the range of 1 mm to 5 mm respectively.

In the electrophoresis chip 200, the form of the sample introduction portion 41, the reagent reservoir 51, the diluent reservoir 58, and the drains 45, 55, 57, 59, and 63 is cylindrical. However, the present invention is not limited thereto. In the present invention, examples of the form of each liquid reservoir include, besides a cylinder, a quadrangular prism, a quadrangular pyramid, a cone, and the like. The form of each liquid reservoir may all be the same or may each be different.

In the electrophoresis chip 200, the width and the depth of the capillary channel for sample analysis 5x are the same as that of the electrophoresis chip 2 shown in FIG. 1.

In the electrophoresis chip 200, with respect to the width and the depth of the reagent measurement channel 56 at the maximum portion of a cross-sectional area, for example, the width is in the range of 0.1 mm to 10 mm and the depth is in the range of 0.1 mm to 10 mm.

In the electrophoresis chip 200, with respect to the width and the depth of the orifice 47, for example, the width is in the range of 1 µm to 200 µm and the depth is in the range of 1 µm to 200 µm, and preferably, the width is in the range of 10 µm to 100 µm and the depth is in the range of 10 µm to 100 µm.

In the electrophoresis chip 200, with respect to the width and the depth of capillary channels except for the capillary channel for sample analysis 5x, the reagent measurement channel 56, and the orifice 47, for example, the width is in the range of 10 µm to 1000 µm and the depth is in the range of 10 µm to 1000 µm, and preferably, the width is in the range of 50 µm to 500 µm and the depth is in the range of 50 µm to 500 µm.

In the electrophoresis chip 200, the maximum thickness of the whole electrophoresis chip 200 is a sum of the thickness of the upper substrate 3a and the lower substrate 3b. The thickness of the upper substrate 3a and the lower substrate 3b is as described above.

The method of manufacturing the electrophoresis chip 200 is not particularly limited and conventionally known methods can suitably be used, for example.

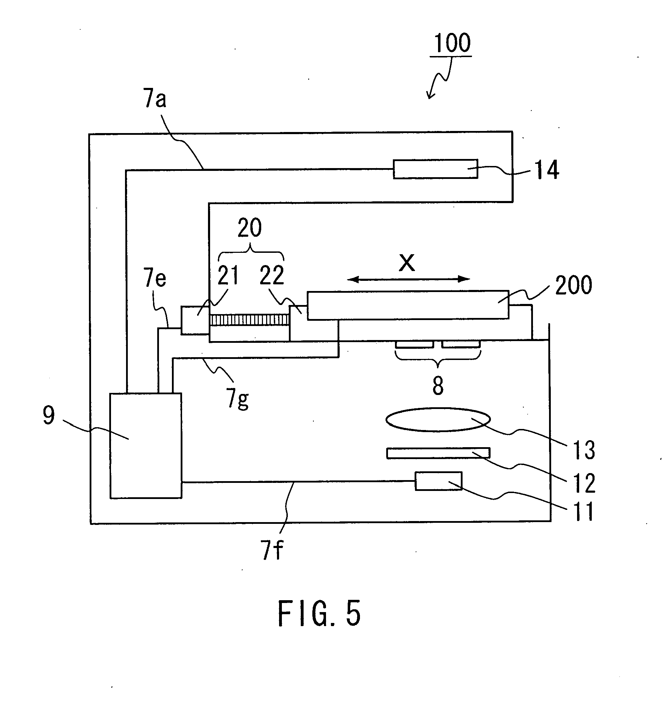

A capillary electrophoresis analysis apparatus 100 of this Example is shown in FIG. 5. In FIG. 5, the portions that are identical to those in FIG. 2 are given the same numbers and symbols. As shown in FIG. 5, the capillary electrophoresis analysis apparatus 100 has the same configuration as the electrophoresis analysis apparatus shown in FIG. 2 except that an electrophoresis chip is the electrophoresis chip 200 shown in FIG. 4 instead of the electrophoresis chip shown in FIG. 1, the quantitative dispensing unit 30, the diluent 31, and the electric wires 7b to 7d are not provided as well as the electrophoresis running buffer is provided in the electrophoresis chip 200 and the connecting portion (not shown) of the connector 70 and an electric wire 7g are provided. Although it is not shown in FIG: 5, the electrophoresis chip 200 is attached to the stage 22 via the connector 70 and disposed in the capillary electrophoresis analysis apparatus 100. Further, the connector 70 is connected to the control unit 9 via the electric wire 7g. The control unit 9 controls power supply or the like to the connector 70.

Next, a method of analyzing protein in blood using the capillary electrophoresis analysis apparatus 100 of this Example is explained.

First, the electrophoresis chip 200 is attached to the capillary electrophoresis analysis apparatus 100 via the connector 70. Next, as described above, the capillary channel for sample analysis 5x is filled with the electrophoresis running buffer. Then, as described above, the electrophoresis running buffer is measured and introduced into the diluent reservoir 58. Further, as the sample, a human whole blood is introduced from the sample introduction portion 41 as described above. Furthermore, as described above, a human whole blood corresponding to the volume of the sample measurement channel 46 is measured and introduced into the diluent reservoir 58. The sample and the electrophoresis running buffer thus introduced are mixed in the diluent reservoir 58 and stirred by rotating the stirring bar (not shown) by a magnetic stirrer (not shown).

Next, the voltage is applied to the electrodes 6a and 6b, thereby creating a potential difference between both ends of the capillary channel for sample analysis 5x. The sample is thereby moved from the electrophoresis starting point 80 to the electrodes 6b side. The voltage application is performed by supplying power from the connector 70 to the electrodes 6a and 6b via the electric wire 7g. The voltage is not particularly limited, however is, for example, in the range of 0.5 to 20kV.

Next, in the same manner as in Example 1, the light is dispersed and collected, and then the detecting point 90 is irradiated with the light (wave length of 415 nm), from which a stray light is further removed. Then, the transmitted light at the detecting point 90 is detected by the detection unit 14 and the absorbance of the protein in blood in the sample that is subjected to the electrophoresis is measured. Electropherogram is made that indicates relationship between degree of the obtained absorbance and analysis time.

The capillary electrophoresis analysis apparatus of the present invention enables the whole apparatus to be small, operation to be simple, manufacturing cost to be inexpensive, and protein in blood to be analyzed highly accurately. Particularly, the capillary electrophoresis analysis apparatus of the present invention is suitable for a micro total analysis systems (µTAS). The present invention is applicable to all technical fields where protein in blood is analyzed, such as laboratory tests, biochemical examinations and medical research. The intended use of the capillary electrophoresis analysis apparatus is not limited and it is applicable to a broad range of technical fields.

## Claims

1. A capillary electrophoresis analysis apparatus for analyzing protein in blood by a capillary electrophoresis method, wherein the capillary electrophoresis analysis apparatus comprises
an electrophoresis chip, a voltage application unit, and an absorbance measurement unit,
the electrophoresis chip comprises a substrate, a plurality of liquid reservoirs, and a capillary channel,
the plurality of liquid reservoirs are formed in the substrate,
the plurality of liquid reservoirs are in communication with one another via the capillary channel,
the capillary channel includes a capillary channel for sample analysis,
the voltage application unit comprises an electrode,
the capillary channel for sample analysis is filled with an electrophoresis running buffer,
a sample containing the protein in blood to be analyzed is introduced into the capillary channel for sample analysis that is filled with the electrophoresis running buffer,
the sample is subjected to an electrophoresis by applying voltage to the electrode, and
an absorbance of the protein in blood in the sample subjected to the electrophoresis is measured by the absorbance measurement unit.

2. The capillary electrophoresis analysis apparatus according to claim 1, wherein in the capillary channel for sample analysis, a cross-sectional shape perpendicular to a channel direction is circular or rectangular,
in a case of circular, a diameter thereof is in a range of 25 µm to 100 µm, and in a case of rectangular, a width thereof is in a range of 25 µm to 100 µm and a depth thereof is in a range of 25 µm to 100 µm.

3. The capillary electrophoresis analysis apparatus according to claim 1, wherein the voltage application unit can adjust the level of voltage when applying the voltage.

4. The capillary electrophoresis analysis apparatus according to claim 1, wherein a spectroscopic method of the absorbance measurement unit is a pre-spectroscopic method.

5. The capillary electrophoresis analysis apparatus according to claim 1, wherein a measurement wave length of the absorbance is in a range of 260 nm to 300 nm or 380 nm to 450 nm.

6. The capillary electrophoresis analysis apparatus according to claim 5, wherein the measurement wave length of the absorbance is in a range of 400 nm to 430 nm.

7. The capillary electrophoresis analysis apparatus according to claim 1, further comprising at least one of a quantitative dispensing unit and a stirring unit.

8. The capillary electrophoresis analysis apparatus according to claim 1, further comprising a liquid sending unit, wherein at least one of the electrophoresis running buffer and the sample is introduced into the capillary channel by the liquid sending unit.

9. The capillary electrophoresis analysis apparatus according to claim 1, further comprising a stray light removing unit.

10. The capillary electrophoresis analysis apparatus according to claim 1, further comprising a position adjustment unit, wherein at least one of a position of the electrophoresis chip and a position of the absorbance measurement unit is adjusted by the position adjustment unit.

11. The capillary electrophoresis analysis apparatus according to claim 1, further comprising a contaminant removal unit, wherein contaminants in the sample can be removed by the contaminant removal unit.

12. The capillary electrophoresis analysis apparatus according to claim 11, wherein the contaminant removal unit is a pre-filter.

13. The capillary electrophoresis analysis apparatus according to claim 1, further comprising:
an air vent unit; and
a detecting point on the capillary channel for sample analysis irradiated with light of a specific wavelength by the absorbance measurement unit, wherein the air vent unit is arranged between the detecting point and the liquid reservoir in which the sample is introduced or stored.

14. The capillary electrophoresis analysis apparatus according to claim 1, further comprising the electrophoresis running buffer.

15. The capillary electrophoresis analysis apparatus according to claim 1, further comprising a diluent.

16. The capillary electrophoresis analysis apparatus according to claim 1, wherein the protein in blood serving as a measurement item is hemoglobin.

17. The capillary electrophoresis analysis apparatus according to claim 16, wherein the hemoglobin is at least one selected from the group consisting of normal hemoglobin, glycated hemoglobin, modified hemoglobin, variant hemoglobin, and fetal hemoglobin.

18. The capillary electrophoresis analysis apparatus according to claim 16, wherein the hemoglobin is at least one selected from the group consisting of hemoglobin A1c, hemoglobin F, hemoglobin A2, hemoglobin S, and hemoglobin C.

19. The capillary electrophoresis analysis apparatus according to claim 16, wherein a concentration of the hemoglobin is measured.

20. The capillary electrophoresis analysis apparatus according to claim 19, wherein the hemoglobin is hemoglobin A1c.

21. The capillary electrophoresis analysis apparatus according to claim 1, wherein the sample is a sample of blood which is subjected to a hemolysis treatment, and the protein in blood is hemoglobin.

22. The capillary electrophoresis analysis apparatus according to claim 21, wherein the hemolysis treatment is at least one selected from the group consisting of a surfactant treatment, an osmotic pressure treatment, and a sonication treatment.
